**Europäisches Patentamt**

**European Patent Office**

**Office européen des brevets**

(19)

(11) Publication number: **0 121 712**
**B1**

# (12) EUROPEAN PATENT SPECIFICATION

(45) Date of publication of patent specification: **22.04.87**

(21) Application number: **84101945.8**

(22) Date of filing: **24.02.84**

(51) Int. Cl.⁴: **C 08 L 1/26,** A 01 N 25/10, A 61 K 9/18, A 61 L 9/04

(54) Sustained release compositions from cellulose ethers.

(30) Priority: **03.03.83 US 471595**
**03.03.83 US 471596**

(43) Date of publication of application:
**17.10.84 Bulletin 84/42**

(45) Publication of the grant of the patent:
**22.04.87 Bulletin 87/17**

(84) Designated Contracting States:
**AT BE CH DE FR GB IT LI NL**

(56) References cited:
**GB-A- 734 624**
**GB-A-1 468 815**
**GB-A-2 082 539**
**US-A-2 404 698**

(73) Proprietor: **THE DOW CHEMICAL COMPANY**
**2030 Dow Center Abbott Road P.O. Box 1967**
**Midland, MI 48640 (US)**

(72) Inventor: **Alderman, Daniel Allison**
**1900 Candlestick Lane**
**Midland Michigan 48640 (US)**
Inventor: **Zweigle, Maurice Laverne**
**908 Crescent Drive**
**Midland Michigan 48640 (US)**

(74) Representative: **Casalonga, Axel et al**
**BUREAU D.A. CASALONGA OFFICE JOSSE &**
**PETIT Morassistrasse 8**
**D-8000 München 5 (DE)**

**Description**

This invention relates to compositions for the sustained release of organic compounds.

The usefulness of many organic compounds, such as insecticides, herbicides, antimicrobials, fertilizers, medicines, fragrances, flavorings, and the like, would be greatly improved if an inexpensive, effective system for the continual release of such compounds were available. Many organic compounds are volatile or unstable in the environment in which they are employed. When large doses of such compounds are used, much of the compound volatilizes or decomposes before performing its desired function. For example, volatile fragrances often exhibit a strong aroma for a relatively short period of time, but then exhibit little or no odor. An equivalent amount of such fragrance could be more effectively employed if small portions thereof were released into the atmosphere over a longer period of time.

Similarly, many flavorings, especially those employed in chewable substances such as chewing gums or chewing tobacco, are advantageously released in continuous, small amounts.

Accordingly, it would be desirable to administer such compounds in a form which reduces the rate of volatilization or decomposition thereof while maintaining effective levels of the compound in the system treated therewith. Other organic compounds are effective in very low concentrations, and are most effectively employed when such low effective concentrations are continually released into the system being treated. Moreover, the use of still other such compounds would be increased if they could be employed as a solid, such as a film or powder.

Various processes are known in which polymeric materials are employed in conjunction with organic compounds to form sustained release systems. For example, in U.S. Patent Nos. 3,795,744 and 3,857,964, it is taught to form a coating of a polymeric material such as a cellulose ether around the organic compound to be released therefrom. The organic compound is released by the physical destruction of the polymeric coating or by the leaching of the organic compound through the polymeric coating. Unfortunately, due to the limitations in size and physical form of such coated compositions, the use thereof is greatly restricted. In addition, the coating operation must be carefully controlled in order to obtain a product having predictable and desirable release characteristics.

GB—A—734624 discloses a process for applying an active material to a cellulose ether which, however, is soluble in cold water.

Another conventionally employed sustained release system comprises forming a solid matrix of a binder material such as a cellulose ether, which matrix has dispersed therein the organic compound to be released. While such matrix systems are somewhat useful in the preparation of pharmaceutical tablets, the size of such matrix systems often precludes the use thereof in other applications. In addition, the active agent is often unevenly distributed in such matrix systems, causing uneven release of the active agent.

Accordingly, an easily prepared sustained release system for organic compounds, which system has a physical form which is amenable to a variety of uses would be highly desired.

The present invention is an aqueous dispersion comprising a plurality of solid particles of a water-insoluble, organophilic cellulose ether, said particles having reversibly diffused therein an active agent which exhibits a greater affinity for the cellulose ether than for the aqueous phase. Under suitable conditions, the active agent diffuses out of the cellulose ether particles, thereby producing a continuous, sustained release of the active agent. The dispersions of this invention can be employed while in the form of a dispersion or may be dewatered using any suitable process to produce a powdery material having sustained release properties. In addition, the dispersions of this invention may be coalesced to form films or other articles which slowly release the active agent.

The cellulose ether employed is one which is insoluble in water and one in which the active agent can become reversibly diffused. In general, the substituent groups on the cellulose ether are chosen such that it renders the cellulose ether somewhat organophilic. Exemplary cellulose ethers useful herein include the alkyl cellulose ethers, especially $C_2$—$C_4$ alkyl cellulose ethers; 2-hydroxyalkylcellulose ethers, especially $C_4$-hydroxyalkylcellulose ethers; mixed alkylhydroxyalkylcellulose ethers, especially $C_2$—$C_4$ alkyl $C_3$—$C_4$ hydroxyalkylcellulose ethers; and the like. In general, both the organophilic character and the water insolubility of these cellulose ethers increase with increasing size of the substituent alkyl or hydroxyalkyl groups, as well as with increasing amounts of such substitution. The manipulation of the amounts and types of substitution to prepare a water-insoluble product is within the skill of those familiar with preparing cellulose ethers, and an exhaustive enumeration of suitable cellulose ethers is not considered to be necessary herein. Most preferred are ethylcellulose, ethylmethylcelulose, ethylhydroxypropylmethylcellulose ethers such as described in European Patent Application Serial No. 83.112486.2, filed 1983 December 12.

The cellulose ether is dispersed as a plurality of finely divided particles into a continuous aqueous phase. Aqueous dispersion of water-insoluble cellulose ethers such as ethylcellulose and methods for their preparation are known in the art and are described, for example, in U.S. Patent Nos. 2,345,879, 4,177,177 and in European Patent Application Serial No. 83.112277.5, filed 1983 December 07. At least sufficient water to form a continuous aqueous phase is employed, and more typically, sufficient water is employed to form a dispersion containing about 5—40 weight percent solids. The aqueous phase may contain, in addition to water, a comiscible or water-soluble organic compound or polymer, which may be present for purposes such as increasing or decreasing the viscosity of the aqueous phase, increasing the volatility thereof,

**0 121 712**

increasing the compatibility thereof with additional, optional components of the dispersion, and the like. The use of such organic compound or polymer is beneficial as long as the active agents therein have a greater affinity for the cellulose ether particles than the aqueous phase. Examples of such organic compound or polymer include thickeners such as polyvinyl alcohol and water-soluble cellulose ethers; water-miscible organic compounds such as acetone, ethanol, methanol, and the like.

The cellulose ether is dispersed into the aqueous phase as a plurality of finely divided particles. While a suspending agent is not necessarily employed if the cellulose ether particles are sufficiently small or the viscosity of the aqueous phase sufficiently high, the preparation of such finely divided cellulose ether particles is difficult and a high viscosity aqueous phase is not generally desirable. Accordingly, a suspending agent is generally employed herein. In general, the choice of suspending agent is not especially critical as long as the dispersion is stabilized therewith, i.e., the dispersed particles do not substantially agglomerate or settle out of aqueous phase. However, the suspending agent must be chose such that it is substantially inert to the active agent employed in the dispersion. For example, certain suspending agents such as those containing weak acid groups, are primarily useful at high pH. Such suspending agents cannot be employed when the active agent is reactive or unstable at high pH. Similarly, suspending agents which are useful at low pH are not suitably employed when the active agent degrades or reacts at such low pH. Exemplary such stabilizers include surfactants, such as alkyl sulfonates and sulfates, alkyl benzyl sulfates and sulfonates, sulfonated condensation products of phenols or alkylphenols with ethylene oxide or propylene oxide, condensation products of long chain aliphatic alcohols with ethylene oxide, polymeric stabilizers such as polyvinyl alcohol, polyethylene glycol, carboxymethylcellulose or carboxymethylmethylcellulose.

The suspending agent, when employed, is generally present in an amount from about 0.5—35 weight percent based on the weight of the dispersed polymer particles.

The dispersions of this invention further contain a quantity of an active agent which is reversibly diffused in the cellulose ether particles. As used herein, the term "active agent" refers to an organic compound or mixture of organic compounds which is to be controllably released by the dispersion. Said active agent is an organic compound or composition which is capable of becoming reversibly diffused within the cellulose particle and which has a greater affinity for the cellulose ether than the aqueous phase. By "reversibly diffused in the cellulose ether particles" is meant that the active agent is associated with the cellulose ether particles in such a manner that under suitable conditions it can be released therefrom in its active form. The nature of the association of the active agent with the cellulose ether particles is not especially critical as long as it is reversible and may be, for example, an absorption, an adsorption, or a dissolution of the active agent into the cellulose ether particles.

Water-insoluble cellulose ethers are compatible with a wide range of organic compounds. Any such organic compound, or mixture of such compounds, which is readily diffused into and retained by the cellulose ether particle, may be employed as the active agent herein. In addition, other organic compounds or mixtures thereof which have only limited compatibility with the cellulose ether, (i.e., those which are not readily diffused into the cellulose ether or which, when diffused therein, rapidly diffuse back out of the cellulose ether), can be used herein if employed in conjunction with a compatibilizing material which increases the compatibility of the organic compound and the cellulose ether. Said compatibilizing agent is an organic compound or polymer which is highly compatible with both the cellulose ether and the active agent, and which (a) enables the active agent to become more readily diffused into the cellulose ether particles, and/or (b) enables the active agent to diffuse out of the cellulose ether particles at a desirable rate under the conditions of use. For example, chlorpyrifos may be desirably employed as the active agent herein, but it is not sufficiently compatible with the cellulose ether to be diffused into the cellulose ether particles in useful quantities. The small quantity of chlorpyrifos which becomes diffused into the cellulose ether particles rapidly diffuses back out. However, when a material such as dibutylphthalate is employed as a compatibilizing material, significant quantities of chlorpyrifos can be diffused into the cellulose ether particles, and the rate of release of the diffused chlorpyrifos is significantly reduced.

Exemplary compatibilizing materials are described hereinafter. The particular choice of compatibilizing material employed, if any, will, of course, depend on the particular active agent employed. In general, compatibility of the active agent with the compatibilizing compound will be within the knowledge of the skilled artisan. Alternatively, the compatibility can often be established by routine experimentation, such as by simply mixing or blending the active agent and the compatibilizing material to see if they form a homogeneous blend. Similarly, optimum amounts of compatibilizing materials employed can be established by simple experiments. In general, however, said compatibilizing agent is advantageously employed in an amount in the range from about 0.5—50, preferably from about 5—30 weight percent based on the weight of the cellulose ether.

The active agent may be, for example, a herbicide, insecticide, nematocide, fungicide, antimicrobial or other biocides, a medication, vitamin, coloring, preservative, fragrance, flavoring, or any other organic compound or mixture of organic compounds which is advantageously controllably released into the system to be treated therewith. As long as the active agent can become reversibly diffused, either alone, in a solution, or with the use of a compatibilizing agent, into the cellulose ether particles, its structure is not especially critical. Suitable active agents range from comparatively simple molecules, like carbon tetrachloride to complex molecules such as vitamins.

3

Exemplary herbicides include, for example, alkanolamine salts of dinitro-o-sec-butylphenol, propylene glycol butyl ethers of 2-(2,4,5-trichlorophenoxy)propanolic acid, chlorinated phenoxy acetic acid and salts or esters thereof, salts of 4-amino-3,5,6-dichloropicalinic acid, as well as many other commercially available herbicides.

Suitable insecticides include, for example, chlorpyrifos-(O,O-dialkyl-O-(3,5,6-trichloro-2-pyridyl) phosphorothioate), and fenchlorphos-(O-O-dialkyl-O-(2,4,5-trichlorophenyl) phosphorothioate).

Suitable fungicides include 1,3-dichloropropene, trichloronitromethane (chloropicrin) and mixtures thereof.

Medications and vitamins may also be employed as the active agent herein. Especially useful are those medications and vitamins which are known to be somewhat unstable in the digestive system and those which are more effectively administered in constant small dosages rather than large intermittent dosages. Of particular interest herein are nitroglycerin and the diverse medications commonly employed in throat lozenges.

Exemplary preservatives include the phenylphenols, chlorinated phenylphenols, chlorinated phenols, cyclopentylphenols and hexamethylenetetraamine-1,3-dichloropropane salt.

Organic flavorings usefully employed herein include the so-called essential oils such as allspice, almond, anise, basil, bay, cardamon, cassia, cinnamon, cherry, clove, ginger, lemon, lime, nutmeg, orange, arrowroot, pepper, peppermint, sage, spearmint, thyme, and wintergreen oils; plant extracts such as vanilla, licorice, coffee, tea, coconut, cherry bark, elm bark, and the like extracts; and artificial flavorings such as anethole, benzyl acetate, cinnamaldehyde, methyl anthranilate, methyl salicylate, citral, menthol, allyl caproate, ethyl methylphenylglycidate, vanillin, ethylbutyrate, diacetyl, eugenol, isoamyl acetate, and the like. Additional flavorings, natural and artificial, are described on pages 463-4 of Rogers and Fischelti, "Flavorings and Spices", *Encyclopedia of Chemical Technology*, 3rd Edition, Vol. 10, published by John Wiley & Sons (1980).

It is understood that the foregoing flavorings are generally employed in solutions in an organic solvent which is typically ethanol or an ethanol-water cosolvent. For the purposes of this invention, liquid flavorings may be employed neat, or as solutions, suspensions, or emulsions in any appropriate solvent, emulsion, or suspension medium. The particular form of the flavoring is not especially critical as long as it is capable of becoming reversibly diffused into the cellulose ether particles, and has greater affinity for cellulose ether particles than for the aqueous phase. Preferably, the flavoring is employed as a solution in alcohol or an alcohol/water cosolvent system.

Fragrances suitably employed herein include any of the commercial perfume and fragrance formulations. Alternatively, the so-called essential oils and the like which are commonly employed in scents, may be used herein. Suitable essential oils include amyris, bay, birch, beechwood, rosewood, camphor, cananga, eucalyptus, geranium, jasmine, labdanum, lavandin, lavender, lemon, lime, mint, neroli, origanum, orris root, patchouli, pine, rose, rosemary, sandalwood and ylang ylang. Additional fragrances include clove leaf oil, orange flower water, tuberose extract, floral aldehydric perfumes, benzoin, castoreum, civet, Maté mimosa, myrrh, oakmoss and violet leaves absolute. Artificial fragrances include the so-called aroma chemical such as acetylated cedarwood terpenes, amylcinnamic aldehyde, amyl salicylate, benzyl acetate, benzyl salicylate, citronellol, coumarin, Galaxolide, hexylcinnamic aldehyde, isobornyl acetate, tinalool, Lyral, musk ambrette, phenethyl alcohol and tetrahydromuguol.

The foregoing fragrances may be employed herein neat, if liquids, or more preferably as solutions in organic solvents, i.e., as resinoids, concretes, absolutes or tinctures, or as present in commercial fragrance formulations. The particular form employed is not particularly critical as long as the fragrance is capable of becoming reversibly diffused into the cellulose ether particles and exhibits a greater affinity for the cellulose ether particles than for the aqueous phase.

The foregoing list of active agents suitably employed herein is not presented as a complete listing thereof and the beneficial use of many other organic compounds as the active agent will be apparent to one skilled in the art having the benefit of the present specification.

Materials suitably employed as a compatibilizing material include phosphate esters such as tri-(2-ethylhexyl)phosphate, tricresylphosphate and triphenylphosphate; phthalate esters such as benzylmethylphthalate, cyclohexylbutylphthalate, dibutylphthalate, dimethylphthalate, diphenylphthalate, diethoxyethylphthalate, and the like: carboxylic acids, fatty acids and salts and esters thereof; fatty alcohols; vegetable oils such as castor oil and corn oil; glycol esters of carboxylic acids and mineral oils. In addition, surfactants such as are described in U.S. Patent No. 4,256,505 to Zweigle, are also suitable compatibilizing materials. Many of the known plasticizers for water-insoluble cellulose ethers are also useful herein as a compatibilizing material.

Any amount of active agent may be employed in the dispersions of this invention as long as the active agent is diffused into the cellulose ether particles and a stable dispersion is maintained. It is understood that the maximum amount of active agent suitably employed will depend somewhat on the particular active agent cellulose ether and compatibilizing materials, if any, employed. In addition, the amount of active agent will depend somewhat on the amount of active agent desirably released and the rate of release thereof by the cellulose ether particles. Thus, the amount of active agent may range, in general, from 0.1 to 200, preferably 5 to 100, more preferably 20 to 50, percent based on the weight of the cellulose ether.

In addition to the foregoing components, a dispersion of this invention may, optionally, contain other

4

# 0 121 712

ingredients as are commonly employed in polymeric dispersions including, for example, flavorings, preservatives, pigments, fillers and the like. For certain uses, such as when the dispersions of this invention are to be formed into films or molded articles, it may be desirable to employ a plasticizer in the dispersion in order to improve the mechanical properties of the cellulose ether. Plasticizers for cellulose ethers are well known and include, for example, esters of phthalic acid, phosphate esters, carboxylic acids including fatty acids and salts and esters thereof. It is noted that such plasticizers are useful as compatibilizing materials as well as to improve the mechanical properties of the cellulose ether. Salts of fatty acids, particularly ammonium salts thereof, are of particular interest because such salts perform the three-fold function of plasticizing the cellulose ether, compatibilizing the active agent with the cellulose ether and stabilizing the dispersion. Because of the need to maintain a high pH in dispersions employing fatty acid salts as stabilizers, however, such fatty acid salt-stabilized dispersions are generally not preferred when the active agent degrades or reacts in an alkaline medium.

In addition to the foregoing optional ingredients, the water-insoluble ether may be employed in conjunction with one or more other polymeric materials which may be employed for their particular beneficial properties. Said other polymers may be any which is compatible with, i.e., miscible with or soluble in the water-insoluble cellulose ether, including water-soluble cellulose ethers, such as methylcellulose, hydroxypropylcellulose, hydroxypropylmethylcellulose, carboxymethylhydroxyethylcellulose polyacrylamides, polyacetates, and diverse cellulose esters.

The dispersions of this invention are advantageously prepared by the simple mixing of the active agent into the previously prepared dispersion of the water-insoluble, organophilic cellulose ether. The amount of active agent employed in preparing the dispersions of this invention is that amount which is desirably diffused into the cellulose ether particles. Upon mixing the active agent with the cellulose dispersion, agitation for a relatively short period, typically about five minutes to six hours, usually is effective to cause the diffusion of the active agent into the cellulose ether particles. Often, mild heating, (i.e., to a temperature of 30°—80°C) of the cellulose ether dispersion is necessary during the mixing of the active agent into the dispersion in order to obtain adequate diffusion of the active agent into the cellulose ether particles.

Once prepared, the dispersions of this invention may be employed in any desirable manner. For example, the dispersion may be sprayed or otherwise applied to the system to be treated with the active agent or may be incorporated with other ingredients (for example, a chewing gum base) to form a final product containing the active agent. Alternatively, the dispersion can be dewatered such as by centrifugation or spray drying to form a powder having sustained release properties. In addition, the cellulose ether particles may be coalesced using methods known in the art to form films or other articles having sustained release properties.

The sustained release dispersions of this invention possess several advantages over conventional sustained release systems. The active agent is readily diffused into the cellulose ether particles. In contrast to matrix type sustained release systems, the active agent is more uniformly distributed throughout the cellulose ether particles. The physical form of the dispersion of this invention allows for a wide variety of uses not available to conventional sustained release systems. In addition, the compositions of the dispersion of this invention are readily adapted to tailor the dispersion for the desired end use.

The manner of use of the dispersion of this invention depends largely on the particular active agent employed and the system to be treated therewith. For example, certain insecticides, such as chlorpyrifos are conveniently sprayed onto fields to remove insects therefrom. However, the chlorpyrifos is volatile and much of the chlorpyrifos evaporates before it penetrates the thatch covering the field. Accordingly, much of the chlorpyrifos is lost without acting on the insects. This loss of chlorpyrifos is decreased by employing a dispersion of this invention containing the chlorpyrifos as the active agent. The dispersion can be sprayed or otherwise applied to the field wet or as a dry powder. Because the chlorpyrifos is diffused into cellulose ether particles, the rate of evaporation thereof is greatly reduced. Moreover, chlorpyrifos-containing cellulose ether particles penetrate thatch more efficiently than chlorpyrifos itself. Thus, more of the chlorpyrifos reaches the areas to be treated and is therefore more effectively employed. Once the chlorpyrifos-containing dispersion of this invention penetrates the thatch, the chlorpyrifos is released by the gradual diffusion of the chlorpyrifos out of the particles and by the breaking down of the cellulose ether particles by enzymes and bacteria which reside in the soil.

The following examples are provided to illustrate the scope of the invention but not to limit the scope thereof. All parts and percentages are by weight unless otherwise indicated. .

Example 1

In this example, an aqueous dispersion containing 20 percent solids, having polymer particles containing 75 weight percent ethylcellulose, and 25 percent dibutylphthalate, is employed. The dispersion is stabilized using Calsoft 240 (an alkylaryl sulfonate salt) and ammonium hydroxide is dissolved into the aqueous phase to a 100-g portion of this dispersion is added 20 g of chlorpyrifos and 60 g of water. The mixture is agitated for 4 hours at 65°C. The product is a stable uniform dispersion.

The rate at which the chlorpyrifos evaporates from the dispersion is determined by the following procedure.

The above-prepared dispersion is divided into 5 equal portions. One such portion is analyzed for chlorpyrifos. The 4 remaining portions are placed into an oven at 60°C for 1 hour, 2 hours, 4 hours and 24

5

## 0 121 712

hours, respectively. Each sample is analyzed for chlorpyrifos after its removal from the oven and the amount of chlorpyrifos lost (based on the chlorpyrifos in the portion which is not heated) is calculated. For comparison, four 12-gram portions of chlorpyrifos are also heated in an oven at 60°C for 1, 2, 4 and 24 hours and the amount of material lost determined for each portion. The results are as given in Table I following.

TABLE I
Percent chlorpyrifos lost at 60°C

| Sample | 0 hour | 1 hour | 2 hours | 4 hours | 24 hours |
|---|---|---|---|---|---|
| Chlorpyrifos | 0 | 17 | 51 | 77 | 100 |
| EC* | 0 | 10 | 45 | 51 | 99 |

*EC is ethylcellulose dispersion.

It can be seen from the foregoing table that the rate of loss of chlorpyrifos from the dispersion of this invention is significantly reduced as compared with the blank sample.

The relative ability of the chlorpyrifos-containing dispersion and pure chlorpyrifos to penetrate thatch is determined by the following procedure.

Five grams of pure long-filtered spaghum moss is soaked in 45 ml of tap water. The wet moss is placed into a funnel over a 4.25 inch (108 mm) diameter, 16 mesh wire screen (1.19 mm openings), producing a thatch layer 15 mm thick. A sample of chlorpyrifos-containing ethylcellulose dispersion prepared as indicated in this example is diluted with water until the dispersion contains 4.5 mg of chlorpyrifos per 0.75 ml of dispersion (6000 ppm). A 0.75-ml portion of this dispersion is then sprayed onto the thatch and allowed to dry at room temperature for 30 minutes. After drying, 100 ml of tap water is sprayed onto the thatch. The water that penetrates the thatch in the first 30 minutes after application of the tap water is collected and analyzed for chlorpyrifos. A second 100-ml portion of tap water is sprayed onto the thatch and the water penetrating the thatch is again collected for 30 minutes and analyzed for chlorpyrifos. The total amount of chlorpyrifos which penetrated the thatch is found to be 2.66 g, or 59 percent of the chlorpyrifos contained in the dispersion sprayed onto the thatch.

For comparison, the experiment is repeated this time using 0.75 ml of a 6000 ppm aqueous solution of chlorpyrifos instead of the aqueous ethylcellulose dispersion. Only 29 percent of the chlorpyrifos is found to penetrate the thatch. Thus, it is seen that thatch penetration is doubled when a chlorpyrifos-containing dispersion of the invention is employed.

Example 2

To 100 g of a 20 weight percent solids aqueous dispersion of particles containing 75 weight percent ethylcellulose and 25 weight percent dibutylphthalate is added 20 g nitrapyrin (a nitrogen stabilizer) and 60 grams water. This mixture is heated with agitation to 70°C for 4 hours, then cooled to room temperature. On inspection, it is seen that all the nitrapyrin is diffused into the dispersed ethylcellulose polymers.

This product is heated at 30°C in an open vessel. After 16 hours of heating, over 80 weight percent of the nitropyrin remains diffused within the ethylcellulose particles. An equivalent amount of pure nitrapyrin completely evaporates under the same conditions.

Example 3

In this example, a dispersion containing particles of 75 weight percent ethylcellulose and 25 weight percent riconoleic acid, stabilized with 3 g octylphenoxy polyethoxyethanol surfactant and adjusted to pH 5.5 with HCl is employed. To 100 g of this dispersion is added 7.5 g of a 20 weight percent dibromonitrilopropionamide (DBNPA) solution in a tetraethylene glycol/water solvent system. The mixture is stirred at room temperature for 72 hours. All the DBNPA is diffused into the polymer particles.

Example 4

In this example, a 30 percent solids aqueous dispersion containing particles containing 75 weight percent ethylcellulose and 25 weight percent oleic acid is employed. The pH of the aqueous phase is adjusted to about 9—10 with ammonia. To 100 g of the ethylcellulose dispersion is added 25 g of lemon oil, USP. The mixture is heated to 70°C and stirred at that temperature for 10 minutes. The resulting dispersion is then cooled. A portion of the resulting dispersion is cast as a film on a sheet of glass and dried at 140°F (60°C). This film has a strong lemon aroma which remains strong for 2 days and is still detectable after standing at room temperature for 6 days. A faint lemon flavor is noticed upon placing a portion of the newly-dried film in one's mouth. The flavor becomes strong on chewing the film. The flavor is still detectable after the film has been standing 6 days at room temperature.

Example 5

A 100-g portion of the ethylcellulose dispersion employed in Example 2 is mixed with 50 g of Avon

6

Everest brand cologne. The mixture is stirred for about 30 minutes at 75°C. A portion of the resulting dispersion is then cast onto paper. For comparison, a sample of Avon cologne as sold commercially is placed onto a separate sheet of paper. After 6 hours, the paper containing the dispersion of this invention had a noticeably stronger aroma than the control sample. After 3 days, the sample of this invention still exhibited a noticeable odor. The control exhibited little or no odor after 6 hours.

Example 6

To a 100-g portion of Aquacoat (Trademark of FMC Corporation) ethylcellulose dispersion is added 50 percent based on weight of the solids of Aquacoat of cherry oil and 25 weight percent based on solids of oleic acid. This mixture was heated at 75°C for 30 minutes. The resulting flavor dispersion is cast as a film on glass and dried at room temperature until the water is evaporated. The resulting film exhibited a sustained flavor and fragrance. This experiment is repeated without adding oleic acid. Without oleic acid, the cherry oil did not diffuse into the Aquacoat.

**Claims**

1. An aqueous dispersion capable of the sustained release of an active agent, said dispersion comprising a plurality of solid particles of a water-insoluble organophilic cellulose ether dispersed into an aqueous phase, said cellulose ether particles having reversibly diffused therein an active agent, said active agent being an organic compound or composition which exhibits a greater affinity for the cellulose ether than aqueous phase.

2. The dispersion of Claim 1 wherein the cellulose ether is ethylcellulose or ethylhydroxypropylmethylcellulose.

3. The dispersion of Claim 1 wherein the active agent is present in an amount from 5 to 100 percent by weight of the cellulose ether.

4. The dispersion of Claim 1 further comprising a compatibilizing material.

5. The dispersion of Claim 2 wherein the aqueous dispersion comprises particles of ethylcellulose or ethylhydroxypropylmethylcellulose, which particles are plasticized with a plasticizing composition comprising a carboxylic acid which is a plasticizer for said cellulose ethers, and the organic phase has a pH which is an alkaline pH useful for dispersions employing fatty acid salts as stabilizers and thus is sufficiently high that sufficient of said carboxylic acid is in salt form to stabilize the dispersion.

6. The dispersion of Claim 5 wherein the carboxylic acid is a fatty acid.

7. A powder containing said active agent, which powder is formed by dewatering the dispersion of Claim 1.

8. A film or other article containing the active agent, which film or other article is formed by the coalescence of the dispersion of Claim 1.

9. The dispersion of Claim 1 wherein the active agent is a biocide.

10. The dispersion of Claim 9 wherein the active agent is chlorpyrifos.

11. The dispersion of Claim 1 wherein the active agent is a medication, vitamin or preservative.

12. The dispersion of Claim 11 wherein the active agent is nitroglycerine.

13. The dispersion of Claim 1 wherein the active agent is a fragrance or flavoring.

14. The dispersion of Claim 13 wherein the flavor or fragrance comprises an essential oil, a plant extract, an aroma chemical or an artificial flavoring.

15. Process for preparing an aqueous dispersion capable of the sustained release of an active agent, which comprises mixing an active agent with a dispersion comprising a plurality of solid particles of a water-insoluble organophilic cellulose ether dispersed into an aqueous phase, with agitation for a period from five minutes to six hours and optionally mild heating to a temperature from 30 to 80°C to obtain adequate diffusion of the active agent into the cellulose ether particles.

**Patentansprüche**

1. Wäßrige Dispersion, geeignet zur kontinuierlichen Abgabe eines aktiven Mittels, dadurch gekennzeichet, daß diese Dispersion eine Vielzahl fester Teilchen eines wasserunlöslichen, organophilen Zelluloseethers, der in einer wäßrigen Phase dispergiert ist, umfaßt, wobei die Zelluloseetherteilchen ein aktives Mittel reversibel diffundiert enthalten, wobei das aktive Mittel eine organische Verbindung oder eine Zusammensetzung ist, die eine größere Affinität für den Zelluloseether als für die wäßrige Phase zeigt.

2. Dispersion nach Anspruch 1, dadurch gekennzeichnet, daß der Zelluloseether Ethylzellulose oder· Ethylhydroxypropylmethylzellulose ist. ·

3. Dispersion nach Anspruch 1, dadurch gekennzeichnet, daß das aktive Mittel in einer Menge von 5 bis 100 Gew.-% des Zelluloseethers vorhanden ist.

4. Dispersion nach Anspruch 1, dadurch gekennzeichnet, daß sie weiterhin ein Kompatibilisierungsmittel enthält.

5. Dispersion nach Anspruch 2, dadurch gekennzeichnet, daß die wäßrige Dispersion Teilchen von Ethylzellulose oder Ethylhydroxypropyl-methylzellulose umfaßt, wobei die Teilchen mit einer Weichmacherzusammensetzung, enthaltend eine Carbonsäure, die ein Weichmacher für die Zellulose-ether ist,

**0 121 712**

weichgemacht wurde und daß die organische Phase einen pH hat, der alkalisch ist und geeignet ist für Dispersionen unter Verwendung von Fettsäuresalzen als Stabilisierungsmittel und der genügend hoch ist, daß genügend Carbonsäure in Salzform vorliegt, um die Dispersion zu stabilisieren.

6. Dispersion nach Anspruch 5, dadurch gekennzeichnet, daß die Carbonsäure eine Fettsäure ist.

7. Pulver, enthaltend ein aktives Mittel, wobei das Pulver durch Entwässern der Dispersion von Anspruch 1 erhalten wurde.

8. Film oder anderer Gegenstand, enthaltend das aktive Mittel, wobei der Film oder der andere Gegenstand gebildet wird durch Zusammenballung der Dispersion von Anspruch 1.

9. Dispersion nach Anspruch 1, dadurch gekennzeichnet, daß das aktive Mittel ein Biozid ist.

10. Dispersion nach Anspruch 9, dadurch gekennzeichnet, daß das aktive Mittel Chlorpyrifos ist.

11. Dispersion nach Anspruch 1, dadurch gekennzeichnet, daß das aktive Mittel ein Medikament, ein Vitamin oder ein Konservierungsmittel ist.

12. Dispersion nach Anspruch 11, dadurch gekennzeichnet, daß das aktive Mittel Nitroglycerin ist.

13. Dispersion nach Anspruch 1, dadurch gekennzeichnet, daß das aktive Mittel ein Duststoff oder ein Aromatisierungsmittel ist.

14. Dispersion nach Anspruch 13, dadurch gekennzeichnet, daß das Aromatisierungsmittel oder der Duftstoff ein etherisches Öl, einen Pflanzenextrakt, ein chemisches Aroma oder einen künstlichen Aromatisierungsstoff umfaßt.

15. Verfahren zur Herstellung einer wäßrigen Dispersion, die geeignet ist, ein aktives Mittel kontinuierlich abzugeben, bestehend aus Mischen eines aktiven Mittels mit einer Dispersion, umfassend eine Vielzahl fester Teilchen eines wasserunlöslichen, organophilen Zelluloseethers, der in einer wäßrigen Phase dispergiert ist, unter Rühren für einen Zeitraum von 5 Minuten bis 6 Stunden und gegebenenfalls mildes Erhitzen auf eine Temperatur von 30 bis 80°C, um eine entsprechende Diffusion des aktiven Mittels in die Zelluloseetherteilchen zu erhalten.

**Revendications**

1. Dispersion aqueuse capable de libérer de façon prolongée un agent actif, ladite dispersion comprenant une pluralité de particules solides d'un éther de cellulose organophile et insoluble dans l'eau, dispersées dans une phase aqueuse, lesdites particules d'éther de cellulose contenant un agent actif dans un état de diffusion réversible, ledit agent actif étant un composé ou une composition organique qui présente une plus grande affinité pour l'éther de cellulose que pour la phase aqueuse.

2. Dispersion conforme à la revendication 1, dans laquelle l'éther de cellulose est de l'éthylcellulose ou de l'éthylhydroxypropylméthylcellulose.

3. Dispersion conforme à la revendication 1, dans laquelle l'agent actif est présent en une quantité comprise entre 5 et 100% en poids par rapport à l'éther de cellulose.

4. Dispersion conforme à la revendication 1, comprenant en outre un matériau de compatibilisation.

5. Dispersion conforme à la revendication 2, dans laquelle la dispersion aqueuse comprend des particules d'éthylcellulose ou d'éthyl hydroxypropylméthylcellulose, particules qui sont plastifiées à l'aide d'une composition de plastification comprenant un acide carboxylique qui est un plastifiant pour lesdits éthers de cellulose, et dans laquelle la phase organique présente un pH qui est un pH alcalin utile pour les dispersions employant des sels d'acides gras en tant que stabilisants, et qui est donc suffisamment élevé pour qu'une quantité suffisante dudit acide carboxylique se trouve sous la forme de sel afin de stabiliser la dispersion.

6. Dispersion conforme à la revendication 5, dans laquelle l'acide carboxylique est un acide gras.

7. Poudre formée par déshydratation de la dispersion conforme à la revendication 1, contenant ledit agent actif.

8. Film ou autre article formé par coalescence de la dispersion conforme à la revendication 1, contenant l'agent actif.

9. Dispersion conforme à la revendication 1, dans laquelle l'agent actif est un biocide.

10. Dispersion conforme à la revendication 9, dans laquelle l'agent actif est le chlorpyrifos.

11. Dispersion conforme à la revendication 1, dans laquelle l'agent actif est un médicament, une vitamine, ou un agent de préservation.

12. Dispersion conforme à la revendication 11, dans laquelle l'agent actif est la nitroglycérine.

13. Dispersion conforme à la revendication 1, dans laquelle l'agent actif est un arôme ou un agent de sapidité.

14. Dispersion conforme à la revendication 13, dans laquelle l'arôme ou l'agent de sapidité comprend une huile essentielle, un extrait de plante, un arôme chimique, ou un agent artificiel de sapidité.

15. Procédé de préparation d'une dispersion aqueuse capable de libérer de façon prolongée un agent actif, qui consiste à mélanger un agent actif avec une dispersion comprenant une pluralité de particules solids d'un éther de cellulose organophile insoluble dans l'eau, dispersées dans une phase aqueuse, sous agitation pendant un laps de temps compris entre 5 minutes et 6 heures, et éventuellement chauffage modéré jusqu'à une température comprise entre 30 et 80°C, afin d'obtenir une diffusion adéquate de l'agent actif dans les particules d'éther de cellulose.